# EUROPÄISCHE PATENTSCHRIFT

(11) **EP 3 081 243 B1**
(45) Veröffentlichungstag und Bekanntmachung des Hinweises auf die Patenterteilung: **26.02.2020**
(21) Anmeldenummer: 16160406.1
(22) Anmeldetag: 15.03.2016
(51) Int. Cl.: A61M 1/02

(54) **VORRICHTUNG ZUR SAMMLUNG UND WEITERVERARBEITUNG VON MENSCHLICHEM BLUTPLASMA**
DEVICE FOR COLLECTING AND FURTHER PROCESSING OF HUMAN BLOOD PLASMA
DISPOSITIF DE COLLECTE ET DE TRANSFORMATION DE PLASMA SANGUIN HUMAIN

(30) Priorität: 14.04.2015 DE 202015101828 U
(43) Veröffentlichungstag der Anmeldung: 19.10.2016
(73) Patentinhaber: Heinz Meise GmbH, 58579 Schalksmühle (DE); Stiftung Zürcher Blutspendedienst SRK, 8952 Schlieren (CH)
(72) Erfinder: Reinert, Dirk, 58644 Iserlohn (DE); Goslings, David, 8049 Zürich (CH)
(74) Vertreter: Patentanwälte Dörner & Kötter PartG mbB

(56) Entgegenhaltungen:
- EP-A1- 0 536 594
- EP-A1- 1 144 025
- DE-U1- 29 603 873
- US-A- 5 128 048
- US-A1- 2004 007 540

## Beschreibung

Die Erfindung betrifft eine Vorrichtung zur Sammlung und Weiterverarbeitung von menschlichem Blutplasma, die einen Poolbeutel und mindestens zwei Sammelbeutel und einen Poolbeutel aufweist, die mittels eines Überleitungsschlauchs miteinander verbunden sind.

Die Gewinnung von Blutplasma erfolgt durch Abtrennung der Blutzellen aus Vollblut, welches Blutspendern entnommen ist. Das Blutplasma wird in der Medizin für eine Vielzahl von Anwendungen benötigt. Es hat daher eine erhebliche Bedeutung, beispielsweise für die Herstellung von Arzneimitteln.

Vorrichtungen zur Sammlung und Weiterverarbeitung von menschlichem Blut oder Blutplasma sind zum Beispiel aus der US 5 128 048 A und der EP 0 536 594 A1 bekannt.

Bei einer üblichen Vollblutspende werden dem Blutspender ca. 500 ml Blut entnommen und in einem Blutbeutel gesammelt. Der Beutel mit dem gesammelten Blut wird anschließend in einer Zentrifuge behandelt, so dass sich das im Vollblut enthaltene Plasma oben im Beutel absetzt. Mithilfe einer Presse wird dann das Plasma aus dem Blutbeutel herausgepresst. Folglich sind die einzelnen gewonnenen Mengen an Plasma relativ gering. Eine Weiterverarbeitung dieser kleinen Mengen ist nicht wirtschaftlich, weshalb Blutplasmen verschiedener Personen zu größeren Mengen zusammengeführt werden. Diesen Vorgang der Zusammenführung bzw. des Mischens von Blutplasma verschiedener Personen wird gemeinhin als "Poolen" bezeichnet. Um beispielsweise eine Menge von 1500 ml Blutplasma zu gewinnen, benötigt man das Plasma aus sechs Vollblutspenden.

Das Plasma kann im Rohzustand verarbeitet werden. Zur Steigerung der Qualität des Plasmas kann es durch weitere Maßnahmen behandelt werden. Hier ist unter anderem die Verwendung von Filtern, beispielsweise von Leukozytenfiltern, bekannt (vgl. bspw. DE 296 03 873 U1). Das Durchfließen des Filters führt zu einer Reduktion der Leukozyten, so genannte Leukozytendepletierung. Das so behandelte Plasma wird dann in Sammelbeutel geleitet und der weiteren Verarbeitung zugeführt.

Grundsätzlich erfüllt das beschriebene Vorgehen die Anforderungen; insbesondere das gewonnene leukozytendepletierte Plasma ist zur Weiterverarbeitung gut geeignet. Das beschriebene Vorgehen weist jedoch den Nachteil auf, dass bei der Filtration Plasmaverluste entstehen, die die Menge an Plasma, welches im Anschluss an die Filtration zur Weiterverarbeitung zur Verfügung steht, bis zu einem Fünftel reduziert. Da Blutplasma nicht unbegrenzt zur Verfügung steht und außerdem teuer ist, ist dieser Verlust unerwünscht.

Hier will die Erfindung Abhilfe schaffen. Der Erfindung liegt die Aufgabe zu Grunde, eine Vorrichtung zur Sammlung und Weiterverarbeitung von menschlichem Blutplasma zu schaffen, bei der der filtrationsbedingte Volumenverlust im Plasma reduziert ist. Gemäß der Erfindung wird diese Aufgabe durch die Merkmale des Patentanspruchs 1 gelöst.

Mit der Erfindung ist eine Vorrichtung zur Sammlung und Weiterverarbeitung von menschlichem Blutplasma geschaffen, welches den filtrationsbedingten Volumenverlust im Plasma wesentlich reduziert. Dies findet seine Ursache darin, dass nur ein Filter zur Anwendung kommt. Die Reduzierung der Anzahl an Filtern führt gleichzeitig zu einer Reduzierung der Plasmaverluste. Hinzu kommt, dass durch die Verwendung nur eines Filters die Filtrationszeit reduziert ist, so dass der gesetzlich vorgeschriebene Zeitrahmen für eine Pool-Filtration nicht überschritten wird. Eine Verlängerung der Filtrationsdauer kann sich beispielsweise dadurch ergeben, dass vermehrt rote Blutzellen im Plasma enthalten sind. Die Verkürzung der Filtrationsdauer reduziert die Gefahr, die gesetzlich vorgeschriebene Dauer für eine Pool-Filtration zu überschreiten.

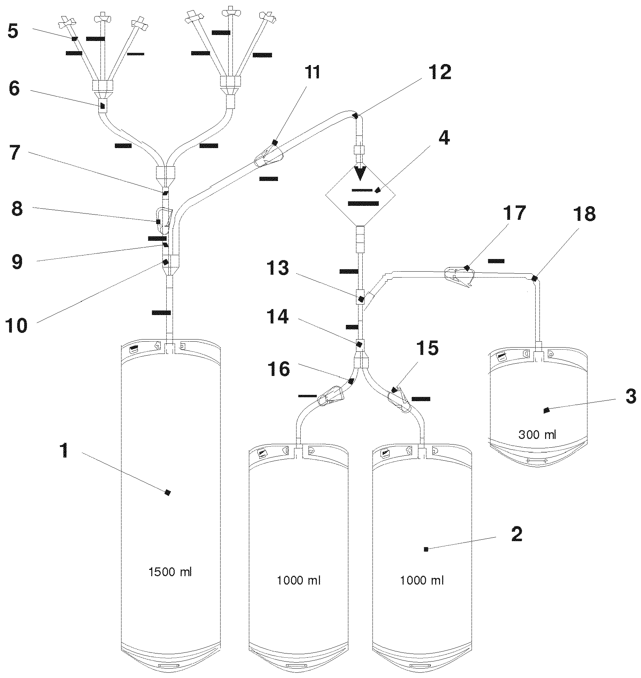

Andere Ausgestaltungen und Weiterbildungen der Erfindung sind in den Unteransprüchen angegeben. Ein Ausführungsbeispiel der Erfindung ist in der Zeichnung dargestellt und wird nachfolgend im Einzelnen beschrieben. Die einzige Figur der Zeichnung zeigt die Darstellung einer Vorrichtung zur Sammlung und Weiterverarbeitung von menschlichem Blutplasma nach der vorliegenden Erfindung.

Die als Ausführungsbeispiel gewählte Vorrichtung zur Sammlung und Weiterverarbeitung von menschlichem Blutplasma weist einen Poolbeutel 1 auf, der im Ausführungsbeispiel ein Fassungsvermögen von 1500 ml hat. An einer Stirnseite des Poolbeutels 1 ist ein Schlauch nicht lösbar angeordnet, der in einen Zweifach-Verteiler 10 mündet. An den Zweifach-Verteiler 10 ist einerseits ein Zulaufschlauch 9, andererseits ein Überleitungsschlauch 12 angeschlossen.

Der Zulaufschlauch 9 ist mit einer Klemme 8 versehen und mündet in einen weiteren Zweifach-Verteiler 7. An den Zweifach-Verteiler 7 sind wiederum zwei Schläuche angeschlossen, die in Dreifach-Verteiler 6 münden, an die jeweils Zulaufschläuche 5 angeschlossen sind. An die Zulaufschläuche 5 sind - nicht dargestellte - Einzelplasmabeutel anschließbar. Das Anschließen erfolgt in der Regel durch steriles Schweißen.

Der Poolbeutel 1 ist mittels des Überleitungsschlauchs 12 mit zwei Sammelbeutein 2 verbunden. Der Überleitungsschlauch 12 ist zudem mit einer Klemme 11 versehen. In dem Überleitungsschlauch 12 ist zwischen dem Poolbeutel 1 und den Sammelbeuteln 2 ein Filter 4 vorgesehen. Am Überleitungsschlauch 12 ist darüber hinaus ein Zweifach-Verteiler 14 vorgesehen, an den zwei Füllschläuche 16 angeschlossen sind, die zu den Sammelbeuteln 2 führen. An den Füllschläuchen 16 sind Klemmen 15 befestigt.

Zwischen dem Filter 4 und dem Zweifach-Verteiler 14 ist ein Y-Verteiler 13 angeordnet, an den über einen Zulaufschlauch 18 ein Überlaufbeutel 3 angeschlossen ist. In dem Zulaufschlauch 18 ist eine Klemme 17 vorgesehen.

Bei der Verwendung der erfindungsgemäßen Vorrichtung werden zunächst die Klemmen 8 und die Klemme 11 geschlossen. Die übrigen Klemmen sind geöffnet. Sodann wird an jeden Zulaufschlauch 5 jeweils ein Einzelplasmabeutel mithilfe eines sterilen Schweißgeräts angeschweißt. Die Vorrichtung nach dem Ausführungsbeispiel ist geeignet, sechs Einzelplasmabeutel anzuschließen. Es ist möglich, durch Verwendung von Mehrfachverteilern anstelle des Zweifach-Verteilers 7 oder Verwendung von mehreren Zweifach-Verteilern 7 eine größere Anzahl von Einzelplasmabeuteln anzuschließen. Auch besteht die Möglichkeit, anstelle des sterilen Schweißens zum Anschließen der Einzelplasmabeutel sterile Kupplungen zu verwenden. Nach dem Anschließen der Einzelplasmabeutel werden diese aufgehängt, so dass der Poolbeutel 1 unterhalb der Einzelplasmabeutel angeordnet ist.

Nach dem Aufhängen der Einzelplasmabeutel wird die Klemme 8 geöffnet. Unter dem Einfluss der Schwerkraft passiert das Plasma die Zulaufschläuche 5 und wird nach Passieren der Dreifach-Verteiler 6 sowie des Zweifach-Verteiler 7 in den Zulaufschlauch 9 überführt. Dort passiert das Plasma den Zweifach-Verteiler 10, bevor es in den Poolbeutel 1 gelangt.

Nach vollständiger Entleerung der Einzelplasmabeutel wird der Zulaufschlauch 9 in unmittelbarer Nähe des Zweifach-Verteilers 10 durchtrennt und gleichzeitig das Ende des Schlauchs verschweißt. Nach dem Durchtrennen sind die Einzelplasmabehälter einschließlich der Schläuche 5 sowie der Verteiler 6 und 7 sowie dem überwiegenden Teil des Zulaufschlauchs 9 von dem Rest der Vorrichtung getrennt. Dieser Teil wird entsorgt.

Im Anschluss wird der Poolbeutel 1 mit einer auf der dem Zulaufschlauch 9 abgewandten Seite des Poolbeutels 1 vorgesehenen Aufhängeöse aufgehängt. In aufgehängtem Zustand sind die Sammelbeutel 2 sowie der Überlauf- bzw. Luftbeutel 3 unterhalb des Poolbeutels 1 angeordnet. Sodann wird die Klemme 11 geöffnet, so dass das in dem Poolbeutel 1 befindliche (gepoolte) Plasma aufgrund der Schwerkraft durch den Filter 4 in Richtung der Sammelbeutel 2 läuft. Soweit es sich bei dem Filter 4 um einen Leukozytenfilter handelt, wird dadurch das Plasma leukozytendepletiert.

Nach vollständigem Entleeren des Poolbeutels 1 hat sich das gefilterte Plasma gleichmäßig auf die Sammelbehälter 2 verteilt. Es wird dann der Überleitungsschlauch 12 zwischen Filter 4 und Y-Verteiler 13 durchtrennt, wobei die Enden des durchtrennten Schlauchs unmittelbar verschweißt werden. Nach dem Abtrennen des Poolbeutels 1 und des Filters 4 werden diese ebenfalls entsorgt.

Die mit dem gefilterten Plasma gefüllten Sammelbeutel 2 werden im Anschluss ausgewogen. Da beide Sammelbeutel 2 den gleichen Inhalt benötigen, der eine vorgegebene Menge nicht überschreiten darf, wird überflüssiges Plasma sowie die überschüssige Systemluft in den Überlaufbeutel 3 gedrückt. Wenn beide Beutel 2 den vorgeschriebenen gleichen Inhalt aufweisen, werden die Klemmen 15 und 17 geschlossen und die Füllschläuche 16 werden in unmittelbarer Nachbarschaft zu den Klemmen 15 abgetrennt, so dass die beiden Sammelbeutel 2 jeweils separat weiterbehandelt werden können. Hierzu werden die Sammelbeutel 2 in nachfolgenden Schritten an weitere Verfahren zur Behandlung des Blutplasmas, beispielsweise zur Herstellung von Arzneimitteln, angeschweißt. Nach der Entleerung der Sammelbeutel 2 am Ende des jeweiligen Verfahrensschritts werden auch diese entsorgt.

Die erfindungsgemäße Vorrichtung zur Sammlung und Weiterverarbeitung von menschlichem Blutplasma stellt eine wesentliche Verbesserung in Bezug auf das Poolen von Blutplasma dar. Zum einen sind aufgrund der Verwendung nur eines Filters, beispielsweise eines Leukozytenfilters, die Verluste an Plasma bis zu einem Fünftel reduziert im Vergleich zu den aus dem Stand der Technik bekannten Vorrichtungen, bei denen die gleiche Anzahl an Filtern Verwendung findet wie Einzelplasmabeutel zum Einsatz kommen. Folglich ist bei gleichbleibend gutem Ergebnis bei der Filtration ein erheblich höherer Wirkungsgrad erzielt.

Zum anderen führt die Verwendung nur eines Filters die Filtrationszeit reduziert ist. Dies bedeutet ebenfalls eine wesentliche Verbesserung im Vergleich zu den aus dem Stand der Technik bekannten Vorrichtungen. Eine Verkürzung der Filtrationszeit ist vorteilhaft, da die Filtrationsdauer gesetzlich beschränkt ist. Da die Filtrationsdauer nicht konstant ist, sondern von den Eigenschaften der zu poolende Plasmaspenden abhängt, konnte es beim Poolen mit den aus dem Stand der Technik bekannten Vorrichtungen zu einem Überschreiten der gesetzlich vorgeschriebenen Dauer kommen. Eine solche Überschreitung Filtrationsdauer hat jedoch zwingend die Vernichtung des gesamten gewonnenen Plasmas zur Folge. Durch die mit Hilfe der erfindungsgemäßen Vorrichtung erzielte Verkürzung der Filtrationsdauer ist dieses Risiko erheblich reduziert.

## Patentansprüche

1. Vorrichtung zur Sammlung und Weiterverarbeitung von menschlichem Blutplasma, die einen Poolbeutel (1) und mindestens zwei Sammelbeutel (2) aufweist, die mittels eines Überleitungsschlauchs (12) miteinander verbunden sind, wobei der Poolbeutel (1) über Zulaufschläuche (5, 9) mit mindestens zwei Einzelplasmabeuteln verbunden ist, und die Vorrichtung nur einen Filter (4) aufweist, der in dem Überleitungsschlauch (12) zwischen dem Poolbeutel (1) und den Sammelbeuteln (2) angeordnet ist.

2. Vorrichtung nach Anspruch 1, **dadurch gekennzeichnet, dass** der Überleitungsschlauch (12) mit einer Klemme (11) versehen ist.

3. Vorrichtung nach Anspruch 1 oder 2, **dadurch gekennzeichnet, dass** im Zulaufschlauch (9) zum Poolbeutel (1) ein Zweifach-Verteiler (10) angeordnet ist, von dem der Überleitungsschlauch (12) abzweigt.

4. Vorrichtung nach einem der vorgenannten Ansprüche, **dadurch gekennzeichnet, dass** am Überleitungsschlauch (12) ein Zweifach-Verteiler (14) vorgesehen ist, an den Füllschläuche (16) zu den Sammelbeuteln (2) angeschlossen sind.

5. Vorrichtung nach Anspruch 4, **dadurch gekennzeichnet, dass** zwischen dem Filter (4) und dem Zweifach-Verteiler (14) ein Y-Verteiler (13) angeordnet ist, an den über einen Zulaufschlauch (18) ein Überlaufbeutel (3) angeschlossen ist.

## Claims

1. Device for collecting and further processing of human blood plasma, which has a pool bag (1) and at least two collection bags (2), which are connected to one another by means of a transfer tube (12), wherein the pool bag (1) is connected to at least two individual plasma bags via inlet tubes (5, 9) and the device has only one filter (4), which is arranged in the transfer tube (12) between the pool bag (1) and the collection bags (2).

2. Device according to claim 1, **characterised in that** the transfer tube (12) is provided with a clamp (11).

3. Device according to claim 1 or 2, **characterised in that** in the inlet tube (9) to the pool bag (1) a dual manifold (10) is arranged, from which the transfer tube (12) branches.

4. Device according to one of the previous claims, **characterised in that** at the transfer tube (12) a dual manifold (14) is provided, to which filling tubes (16) to the collection bags (2) are connected.

5. Device according to claim 4, **characterised in that** between the filter (4) and the dual manifold (14) a Y-manifold (13) is arranged, to which an overflow bag (3) is connected via an inlet tube (18).

## Revendications

1. Dispositif de collecte et de transformation de plasma sanguin humain, présentant une poche de pool (1) et au moins deux poches de collecte (2) reliées entre elles au moyen d'un flexible de transfert (12), sachant que la poche du pool (1) est reliée via des flexibles d'arrivée (5, 9) à au moins deux poches à plasma individuelles, et que le dispositif ne présente qu'un filtre (4) disposé dans le flexible de transfert (12) entre la poche du pool (1) et les poches de collecte (2).

2. Dispositif selon la revendication 1, **caractérisé en ce que** le flexible de transfert (12) est muni d'une pince (11).

3. Dispositif selon la revendication 1 ou 2, **caractérisé en ce que** dans le flexible d'arrivée (9) à la poche du pool (1) est disposé un double distributeur (10) d'où part le flexible de transfert (12).

4. Dispositif selon l'une des revendications précédentes, **caractérisé en ce que** contre le flexible de transfert (12) est prévu un double distributeur (14) auquel sont raccordés des flexibles de remplissage (16) aboutissant aux poches de collecte (2).

5. Dispositif selon la revendication 4, **caractérisé en ce qu'**entre le filtre (4) et le double distributeur (14) est disposé un distributeur en Y (13) auquel est raccordée, via un flexible d'arrivée (18), une poche de débordement (3).
